# FASCICULE DE BREVET EUROPEEN

(11) **EP 0 565 395 B1**
(45) Date de publication et mention de la délivrance du brevet: **02.01.1997**
(21) Numéro de dépôt: 93400703.0
(22) Date de dépôt: 19.03.1993
(51) Int. Cl.: A61F 2/02

(54) **Filtre sanguin perfectionné éventuellement résorbable**
Blutfilter, gegebenenfalls resorbierbares
Blood filter optionally resorbable

(30) Priorité: 07.04.1992 FR 9204226
(43) Date de publication de la demande: 13.10.1993
(73) Titulaire: B. BRAUN CELSA, 86361 Chasseneuil Cédex (FR)
(72) Inventeur: Cottenceau, Jean-Philippe, F-92160 Antony (FR); Chevillon, Gérard, F-91120 Montrouge (FR); Roussigne, Maurice, F-86000 Poitiers (FR); Nadal, Guy, F-86000 Poitiers (FR)
(74) Mandataire: Lerner, François

(56) Documents cités:
- EP-A- 0 420 541
- EP-A- 0 423 916
- FR-A- 2 567 405
- FR-A- 2 580 504

## Description

L'invention a pour objet un filtre sanguin perfectionné destiné à être placé ou implanté dans un vaisseau d'un système circulatoire, afin d'y retenir d'éventuels caillots de sang.

Des filtres de ce type sont par exemple décrits dans les brevets US-A-3 952 747, EP-A-293 605 ou encore EP-A-188 927.

De façon générale, ces filtres se présentent sous la forme d'un petit panier tronconique qui s'accroche à l'intérieur du vaisseau où il est implanté, en aval du trajet que l'on désire filtrer ; il s'agit en général de la veine cave arrivant au coeur.

On peut ainsi arrêter, avant leur entrée dans le coeur, les éventuels caillots sanguins pouvant se former et risquant d'entraîner notamment des embolies.

Une difficulté propre à ce genre d'intervention consiste à positionner correctement le filtre. D'une façon générale, pour introduire un tel filtre dans le vaisseau, on l'y pousse au moyen d'un tube qui traverse ledit vaisseau et dont le diamètre est inférieur à celui de ce dernier. Lorsque le filtre arrive à l'extrémité du tube d'introduction, il est alors lâché dans le vaisseau et l'expansion de ses pattes souvent munies de crochets assure son ancrage.

Un tel "lâché" est dans la pratique très délicat à contrôler, et il s'est avéré dans un certain nombre de cas que le filtre en panier occupait en fait, à l'intérieur du vaisseau, une position autre que la position la plus favorable avec son axe sensiblement parallèle à l'axe du vaisseau.

L'invention, objet du brevet EP-A-188 927 apportait une première solution à ce problème de centrage, en prévoyant que les pattes du filtre seraient pourvues, vers leur extrémité libre, d'appendices orientés sensiblement parallèlement à la paroi cylindrique engendrée par une ligne génératrice parallèle à l'axe de la corolle conique du filtre.

Bien entendu, depuis l'invention de ce filtre, les recherches ont continué.

Au cours de celles-ci, il s'est en particulier avéré qu'il pouvait être intéressant, de pouvoir dans certains cas et si nécessaire, rendre résorbable au moins la partie effectivement filtrante du filtre (c'est-à-dire sa partie s'étendant jusque vers le centre du vaisseau), en maintenant éventuellement non résorbable la partie la plus extérieure d'accrochage au vaisseau, tout en assurant toujours le meilleur centrage possible du filtre.

Les filtres connus en forme de panier tronconique se sont alors avérés présenter certains inconvénients liés notamment au fait que si l'on souhaitait rendre résorbable la partie centrale filtrante, les parties périphériques servant à l'accrochage risquaient de se retrouver, après résorption de cette partie centrale, non liées entre elles avec en conséquence des risques importants de désaccrochage, avec les inconvénients que l'on imagine.

L'invention a pour objet de résoudre ces difficultés en proposant un filtre sanguin perfectionné autocentrable, conçu pour être éventuellement au moins en partie résorbable biologiquement, et comprenant pour cela les caractéristiques de la revendication 1.

Avantageusement, le fil en zigzag présentera une suite de lignes reliées par des portions d'extrémité courbées et pourra occuper élastiquement une première position repliée sous contrainte, dans laquelle lesdites lignes s'étendront sensiblement côte à côte, les unes le long des autres, pour autoriser l'introduction du filtre dans le système circulatoire, et une seconde position déployée où lesdites lignes s'écarteront angulairement les unes des autres pour définir une surface tubulaire sensiblement cylindrique adaptée au vaisseau récepteur.

Selon une autre caractéristique préferée de l'invention, la structure extérieure de maintien du filtre s'étendra exclusivement à la périphérie de ce dernier, la liaison entre cette structure et la partie centrale de tamisage étant située à la périphérie de cette dite partie.

Ainsi si, comme le prévoit une caractéristique complémentaire de l'invention, la partie centrale de tamisage est réalisée en une matière résorbable biologiquement, les risques de migration de la structure extérieure (supposée alors non résorbable) seront très limités, compte tenu de la forme de cette structure, et cela d'autant plus que ses lignes en zigzag devraient venir sensiblement au contact du vaisseau lorsque le filtre sera implanté.

Concernant la conformation de la partie centrale filtrante ou de tamisage, celle-ci pourra notamment être réalisée à partir d'un filament ou d'une série de filaments relativement souples, agencés par exemple pour constituer un filet maillé ou encore de manière que ces filaments se réunissent ou s'imbriquent à la manière d'un faisceau dans une zone sensiblement centrale du filtre.

En particulier, si cette partie de tamisage doit être résorbable, on pourra utiliser des filaments de sections différentes pour réduire vers le centre du vaisseau la résistance à la résorption.

La partie centrale en question pourrait toutefois également se présenter sous la forme d'une étoile monobloc à plusieurs branches réunies entre elles vers le centre de l'étoile, avec même éventuellement des ramifications complémentaires pour la liaison intermédiaires des branches.

On va maintenant donner une description plus détaillée de l'invention, en faisant pour cela référence aux dessins d'accompagnement donnés uniquement à titre d'exemples non limitatifs et dans lesquels :
La figure 1 est une vue schématique d'un filtre conforme à l'invention implanté dans un vaisseau,
la figure 2 est une vue de détail du crochet correspondant au repère II de la figure 1,
la figure 3 est une vue du filtre de la figure 1 seul dans son état expansé mais non implanté,
la figure 4 est une vue de dessous du filtre de la figure 3 dans le sens de la flèche IV,
la figure 5 est une variante de réalisation du filtre,
la figure 6 est une vue de dessous du filtre de la figure 5, dans le sens de la flèche VI,
la figure 7 est une autre variante de réalisation du filtre,
la figure 8 est une vue de dessous de ce même filtre, dans le sens de la flèche VIII de la figure 7,
la figure 9 est une variante de réalisation de la partie de tamisage du filtre de la figure 8,
la figure 10 est une autre variante de réalisation d'une partie de tamisage utilisable sur un filtre de l'invention,
les figures 11 et 12 montrent deux variantes possible de réalisation des yeux d'extrémité de la structure en zigzag et leur liaison avec la partie de tamisage,
et les figures 13 à 15 montrent schématiquement, en trois étapes caractéristiques successives, une possible mise en place d'un type de filtre sanguin conforme à l'invention.

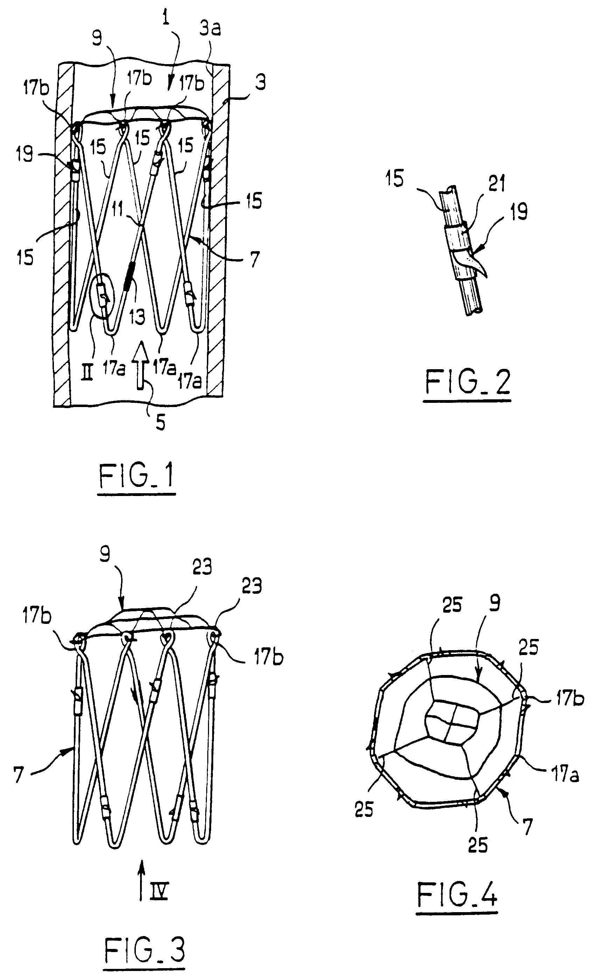

En se référant tout d'abord à la figure 1, on voit donc illustré un filtre 1 ici implanté en position déployée à l'intérieur d'un vaisseau 3 de manière à pouvoir intercepter les éventuels caillots pouvant y circuler, le sens du flux sanguin ayant été repéré par la flèche 5.

Conformément à l'invention, le filtre 1 comprend une partie centrale de tamisage 9 destinée à la retenue d'éventuels caillots et liée périphériquement à la structure extérieure 7 qui est auto-expansible (ou extensible) pour, une fois le filtre lâché, maintenir ce dernier centré sensiblement à son endroit d'implantation.

Comme on peut le constater, la structure de maintien 7 est constituée à cet effet d'au moins un fil relativement rigide 11, conformé en zigzag et enroulé sur lui-même pour présenter une configuration fermée définissant, en l'espèce, une surface tubulaire sensiblement cylindrique plaquée contre la paroi intérieure 3a du vaisseau 3 qui peut être par exemple la veine cave.

En 13, on a représenté la zone de fermeture du fil en zigzag où ses deux extrémités se rejoignent, les repères 15 et 17a, 17b désignant respectivement les suites de lignes et les portions d'extrémités courbées du fil.

Ainsi conformée, cette structure de maintien ne s'étendra donc qu'à proximité immédiate de la paroi du vaisseau, sans quasiment interférer avec l'espace réservé au cheminement du sang, les lignes 15 de la structure devant a priori venir sensiblement au contact de la paroi 3a.

Une telle disposition limite naturellement les risques de migration du filtre.

Toutefois, il est conseillé de prévoir en outre des moyens d'accrochage, tels que repérés 19, devant pénétrer légèrement dans la paroi du vaisseau. Ces moyens d'accrochage peuvent notamment consister chacun en un crochet faisant saillie à une plaque 21 soudée à l'une des lignes 15, comme cela est illustré sur la figure 2. Les crochets rapportés en question pourront notamment s'étendre deux à deux à contre-sens, alternativement d'une ligne à l'autre, de manière à prévenir toute translation du filtre dans un sens ou en sens inverse.

Dans la technique, une telle structure en zigzag, (éventuellement pourvue de tels moyens d'accrochage complémentaires) a en fait déjà été utilisée pour réaliser des élargisseurs de vaisseaux couramment appelés "stents" et employés en tant que moyen de traitement de la sténose (maladie liée au rétrécissement des vaisseaux sanguins, créant des problèmes de circulation grave tels notamment qu'athérosclérose, phlébites...).

Une méthode de traitement de ces sténoses consiste à installer à l'intérieur des veines ou artères un ballon que l'on gonfle pour écarter localement le vaisseau rétréci, après quoi un stent est installé à cet endroit, le stent jouant alors le rôle d'élargisseur ou d'étai de renfort pour empêcher le vaisseau de se rétrécir à nouveau à l'endroit où le dispositif est posé, une autre fonction étant de maintenir plaquée sur elle-même une partie de la paroi du vaisseau qui a pu s'en détacher, ou encore de prévenir l'occlusion future du vaisseau suite à la progression de la maladie athéromateuse.

Aucune de ces fonctions n'est assurée par le filtre sanguin de l'invention ni en particulier par sa structure de maintien 7 dont le rôle est de servir de moyen de maintien du filtre en position, une fois implanté, et de support des moyens d'accrochage 19, en assurant concomitamment le maintien de la partie centrale de tamisage 9.

En d'autres termes, comme tout filtre sanguin, celui de l'invention ne peut en aucun cas être confondu avec un "stent", leurs domaines d'application étant d'ailleurs différents : affection sanguine pour le filtre (possible migration de caillots) et altération d'une paroi de vaisseau (sténose) pour le stent.

Pour en revenir plus spécifiquement au filtre de l'invention, on se reportera maintenant plus particulièrement aux figures 2 et 3 où l'on retrouve le filtre de la figure 1 ici représenté non implanté mais toujours dans son état expansé ou déployé.

En complément de ce qui a été déjà dit, on notera sur ces figures que les portions d'extrémités recourbées 17b de la structure 7 en zigzag présentent ici chacune un oeil ou boucle 23 pour la fixation périphérique de la partie centrale de tamisage 9.

En l'espèce, cette partie 9 se présente sous la forme d'un filet ou d'une "toile d'araignée" réalisée en un ou plusieurs filaments déformables, liés entre eux pour constituer un tel filet maillé, lequel est lié périphériquement à la structure 7 à l'endroit des yeux 23, par exemple par des noeuds (voir figure 4).

En particulier, un tel filet pourra être réalisé à partir d'un filament habituellement employé pour les sutures chirurgicales.

Tandis qu'il est a priori considéré comme peu approprié d'utiliser une matière résorbable pour le fil "rigide" devant constituer la structure en zigzag 7 (une structure en "phynox"-marque déposée-ou encore en acier inoxydable, voire en titane, étant alors conseillée), il est par contre tout à fait envisageable d'utiliser des filaments de suture résorbables pour réaliser le filet 9.

On pourra alors par exemple employer des monofilaments d'acide polyglycolique tressés ou encore d'un copolymère de celui-ci.

A titre de variante, le brevet FR-A-2 635 966 enseigne la possible utilisation de fibres à base de poly-p-dioxanone et de polyglactine.

Si par contre on préfère des fils de suture non résorbables, on pourra alors employer des matières allant du filament métallique aux matières synthétiques (polyester, polyamide), voire des fils de soie naturelle et de lin.

Si toutefois la solution d'une partie de tamisage 9 résorbable est retenue, il est alors conseillé de prévoir une résistance à la résorption biologique de cette partie de tamisage, une fois le filtre implanté, plus faible vers une zone sensiblement centrale de celle-ci qu'à sa périphérie où ladite partie de tamisage est liée à la structure 7.

Ainsi, le filtre ne perdra que progressivement son pouvoir filtrant, cette période s'étendant sur plusieurs mois, voire plusieurs années.

On pourra notamment obtenir cet effet en réalisant le filet 9 avec des mailles plus petites vers le centre que vers la périphérie et/ou en prévoyant des filaments de sections différentes pouvant aller en se rétrécissant à mesure qu'on s'approche du centre du filet.

A titre d'exemple, de tels fils pourraient avoir des diamètres compris entre 0,7 à 5 dixièmes de millimètres.

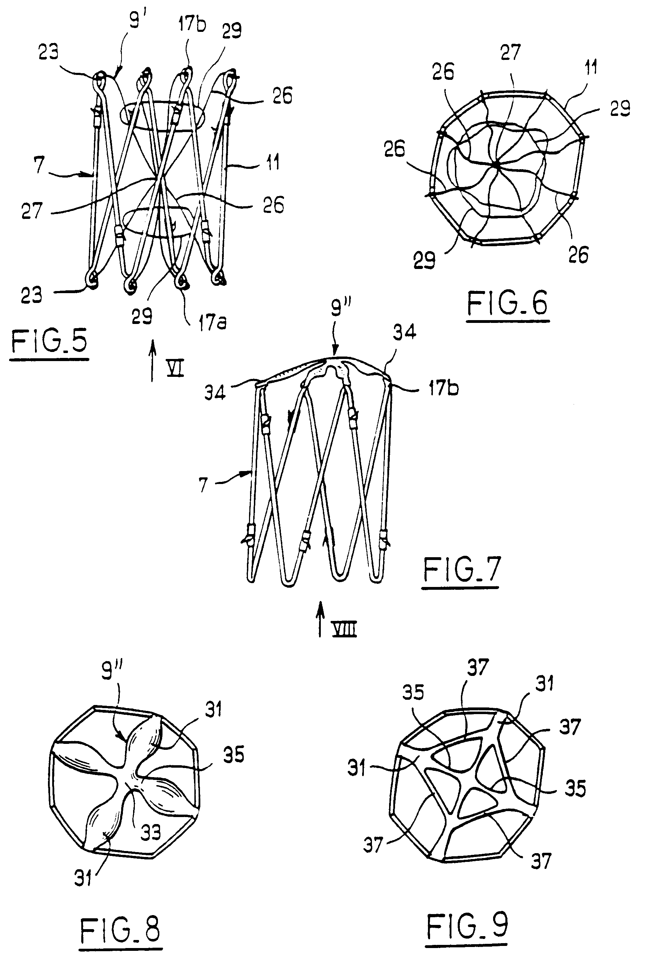

Sur la figure 5, on a illustré une autre forme de réalisation du filtre de l'invention.

En fait, cette variante ne se distingue de la précédente essentiellement que sur deux points : d'une part la structure 7 présente maintenant des yeux 23 à l'une et l'autre de ses deux extrémités opposées 17a, 17b, et la partie centrale de filtrage, ici repérée 9', est maintenant constituée par une série de filaments (résorbables ou non comme précédemment) liés à ladite structure 7 (par exemple noués) à l'endroit des yeux 23, ceci à l'une/ou l'autre desdites extrémités 17a, 17b, les filaments étant agencés pour se réunir ou s'imbriquer, à la manière d'un réseau ou d'un faisceau 26, dans une zone sensiblement centrale du filtre, comme cela apparaît clairement si l'on compare les figures 5 et 6 où la partie centrale d'imbrication a d'ailleurs été repérée 27.

Ainsi, la partie tamisante centrale 9' présentera en quelque sorte une configuration en double tronc de cône inversé occupant le volume central du filtre délimité extérieurement par le fil en zigzag 11.

Comme précédemment, les filaments du tamis 9' pourront être résorbables par voie biologique et présenter des diamètres différents.

Eventuellement, le faisceau de fils 26 pourra être compléter par un ou deux cerclages de fils tels que 29 de section réduite, entourant localement à deux niveaux intermédiaires lesdits fils "principaux" 26 , de manière à augmenter ainsi au moins temporairement le pouvoir filtrant du filtre.

Les figures 7 et 8 illustrent encore une autre variante dans laquelle la structure 7 en zigzag est maintenant dépourvue d'yeux d'extrémités, la partie centrale tamisante, maintenant repérée 9'', se présentant sous la forme d'une étoile monobloc (voir figure 8) souple à plusieurs branches 31 réunies entre elles vers le centre de l'étoile, en 33, et pouvant être par exemple collées périphériquement à des rebords d'extrémités 34, à l'endroit des portions courbées de liaison 17b du filtre.

En particulier dans le cas où cette "étoile de filtrage" sera réalisée en une matière résorbable, les branches présenteront localement, à proximité du centre 33, une section réduite telle que repérée en 35 sur la figure 8 pour l'une des branches.

En pratique, l'étoile en question pourra être notamment réalisée par moulage, découpage ou poinçonnage à partir par exemple d'une feuille ou une fine plaque en matière appropriée. Bien entendu, les branches pourront être très fines.

Si l'étoile doit être résorbable biologiquement, on pourra alors notamment utiliser l'acide polyglycolique ou un acide polylactique.

Et pour réduire localement la section de cette étoile, comme en 35, on pourra obtenir cet effet tant en réduisant sa largeur qu'en réduisant son épaisseur. On pourra alors utiliser une plaque présentant d'un côté une surface concave.

Eventuellement, les branches 31 pourraient être en outre reliées deux à deux par des ramifications intermédiaires transversales telles que repérées 37 sur la figure 9, s'étendant entre le centre et la périphérie de l'étoile.

Bien entendu, d'autres formes en étoile que celles des figures 8 et 9 pourraient être retenues.

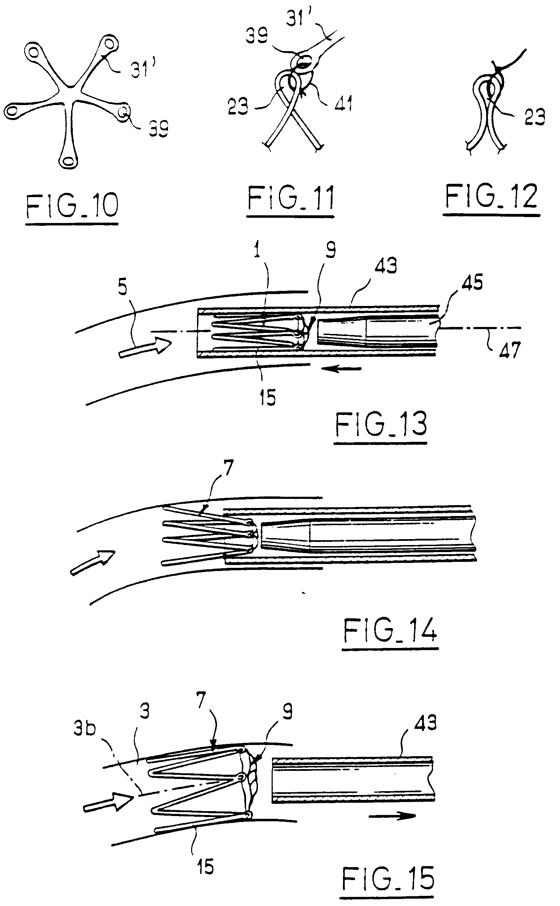

Ainsi, la forme de la figure 10 est envisageable. Alors que les branches de l'étoile de la figure 8 sont d'abord renflées vers la périphérie avant de se restreindre en 35, les branches 31' de l'étoile de la figure 10 vont directement en se rétrécissant depuis la périphérie. Par contre, leur nombre est plus important. En outre, ces branches sont perçées en 39 à leur extrémité libre élargie de manière à présenter là un orifice pouvant être traversé par un lien (tel qu'un filament de suture non résorbable) pouvant être noué chacun dans l'un des yeux 23 que présenterait alors la structure 7 à l'extrémité correspondante, comme cela est illustré sur la figure 11.

A la figure 12, on a illustré une vue de détail montrant au même endroit la fixation d'un filament pouvant être utilisé pour le filtre des figures 3 et 5.

Sur les figures 11 et 12, on remarquera également deux formes possibles de réalisation des yeux 23 : avec croisement (figure 11), ou avec simple écrasement ou étranglement local, sans croisement ni chevauchement (figure 12).

Sur les figures 13 et suivantes, on a illustré schématiquement un possible mode d'introduction du filtre de l'invention.

La mise en place du filtre est faite par voie percutanée (jugulaire ou fémorale habituellement), à travers un tube d'introduction repéré 43 et dénommé habituellement dans la technique "Desilet".

Le filtre 1 est poussé à l'intérieur du tube 43 par le poussoir 45.

On remarquera qu'en place dans le tube, le filtre occupe alors élastiquement sa position repliée sous contrainte, dans laquelle les lignes 15 de son fil en zigzag s'étendent sensiblement côte à côte les unes le long des autres, plus ou moins parallèlement à l'axe 47 du tube.

On notera en outre que le filtre a été placé de manière que sa partie tamisante 9 sorte en dernier, le tube 43 ayant été introduit à contre-sens du flux sanguin (flèche 5).

A la figure 14, le filtre apparaît avec sa structure 7 sortant déjà du tube en présentant une forme sensiblement en corolle.

Sur la figure 15, on aperçoit le filtre un instant plus tard, entièrement déployé, avec sa structure 7 totalement libérée. Le fil en zigzag est alors élastiquement déployé en définissant une enveloppe sensiblement cylindrique quasiment coaxiale à l'axe 3b du vaisseau, l'écartement élastique des lignes du fil entraînant l'accrochage du filtre et le déploiement corollaire de la partie centrale filtrante 9 qui va pouvoir alors parfaitement jouer son rôle de filtrage.

Le tube 43 peut alors être retiré par sa voie d'accès.

Il est clair que les variantes de réalisation du filtre des figures 5 à 10 peuvent être introduites de la même manière.

Concernant la réalisation de la structure 7, on notera juste qu'elle peut aisément être obtenue à partir d'un fil en matière adaptée, conformé à plat en zigzag, puis refermé sur lui-même pour rejoindre ses deux extrémités de manière à définir alors une sorte d'enveloppe sensiblement cylindrique dans son état expansé transversalement ou radialement à son axe de cylindre, cette enveloppe pouvant être obtenue à partir par exemple d'un fil rond en acier d'un diamètre de quelques dixièmes de millimètre, notamment 4 à 8 dixièmes.

## Revendications

1. Filtre sanguin implantable dans un vaisseau (3), sur le trajet sanguin, en particulier pour la retenue de caillots de sang, caractérisé en ce qu'il comprend :
- une structure extérieure (7) auto-expansible, comportant au moins un fil (11) relativement rigide conformé en zigzag et enroulé sur lui-même pour présenter une configuration fermée définissant extérieurement une surface tubulaire sensiblement cylindrique dans l'état expansé de la structure transversalement à son axe de cylindre,
- une partie centrale de tamisage (9, 9', 9'') s'étendant vers l'intérieur de ladite structure et liée audit fil (11) en différents points, pour la retenue d'éventuels caillots, et
- des moyens d'accrochage (19) portés par ladite structure (7), pour l'accrochage du filtre au vaisseau.

2. Filtre sanguin selon la revendication 1 caractérisé en ce que ledit fil (11) conformé en zigzag présente une suite de lignes (15) reliées par des portions d'extrémités courbées (17a, 17b) et peut occuper élastiquement une première position repliée sous contrainte dans laquelle lesdites lignes s'étendent sensiblement côte à côte les unes le long des autres, pour autoriser l'introduction du filtre dans le système circulatoire, et sa dite position expansée où lesdites lignes (15) s'écartent angulairement les unes des autres pour définir ladite surface tubulaire sensiblement cylindrique.

3. Filtre selon la revendication 1 ou la revendication 2 caractérisé en ce que la structure extérieure (7) s'étend exclusivement à la périphérie du filtre, la liaison entre cette structure et la partie centrale de tamisage (9) étant située à la périphérie de cette dernière.

4. Filtre selon l'une quelconque des revendications précédentes caractérisé en ce que la partie centrale de tamisage (9) est en matériau résorbable biologiquement lorsque le filtre est implanté, la résistance à la résorption biologique de cette partie de tamisage étant plus plus faible vers une zone (35) sensiblement centrale de celle-ci qu'elle l'est à sa périphérie où ladite partie de tamisage est liée à la structure extérieure en zigzag.

5. Filtre selon l'une quelconque des revendications 2 à 4 caractérisé en ce que lesdites lignes (15) de la structure extérieure en zigzag sont prévues pour venir sensiblement au contact d'une paroi (3a) du vaisseau lorsque le filtre est implanté.

6. Filtre selon l'une quelconque des revendications précédentes 2 à 5, caractérisé en ce que lesdits moyens d'accrochage sont disposés sur certaines au moins desdites lignes (15) du fil (11).

7. Filtre selon la revendication 6 caractérisé en ce que les moyens d'accrochage consistent en des crochets (19) rapportés sur lesdites lignes (15) du fil en zigzag par l'intermédiaire de plaques (21), ces crochets s'étendant deux à deux à contre-sens pour prévenir la translation du filtre sur le trajet veineux dans un sens ou en sens inverse.

8. Filtre selon l'une quelconque des revendications précédentes caractérisé en ce que ladite partie de tamisage est constituée à partir d'un filament ou de série de filaments relativement souple(s), agencé(s) pour constituer un filet maillé (9) avec des mailles plus petites vers le centre que vers la périphérie où ledit filet est lié à la structure extérieure (7) à fil en zigzag.

9. Filtre selon l'une quelconque des revendications 1 à 7 caractérisé en ce que ladite partie de tamisage (9, 9') est constituée à partir d'un filament ou d'une série de filaments relativement souple(s) d'une section allant en diminuant depuis la périphérie vers le centre de cette partie de tamisage.

10. Filtre selon l'une quelconque des revendications 1 à 7 caractérisé en ce que ladite partie de tamisage (9') comporte une série de filaments relativement souples (26) s'étendant à l'intérieur de ladite structure (7) à fil en zigzag entre les deux extrémités axiales opposées (17a, 17b) de cette dernière vers lesquelles les filaments sont liés à la structure, ces filaments étant agencés pour se réunir ou s'imbriquer à la manière d'un faisceau dans une zone sensiblement centrale (27) du filtre.

11. Filtre selon la revendication 10 caractérisé en ce que, entre les deux extrémités opposées (17a, 17b) de la structure extérieure (7), le faisceau de fils (26) est complété par au moins un cerclage d'un fil complémentaire (29) entourant localement lesdits fils en faisceau (26), pour augmenter au moins temporairement le pouvoir filtrant de ladite partie de tamisage (9').

12. Filtre selon l'une quelconque des revendications 1 à 7 caractérisé en ce que la partie de tamisage (9'') présente la forme d'une étoile monobloc à plusieurs branches (31, 31') réunies entre elles vers le centre (33) de l'étoile.

13. Filtre selon la revendication 12 caractérisé en ce que lesdites branches (31, 31') présentent localement, à proximité du centre (33) de l'étoile, une section réduite.

14. Filtre selon la revendication 12 ou la revendication 13 caractérisé en ce que lesdites branches (31) présentent des ramifications transversales (37) qui relient entre elles deux branches successives, entre le centre (33) et la périphérie de l'étoile.

15. Filtre selon l'une quelconque des revendications 12 à 14 caractérisé en ce que ladite étoile (9'') est réalisée par moulage, découpage ou poinçonnage.

16. Filtre selon l'une quelconque des revendications 12 à 15 caractérisé en ce que ladite étoile (9'') est réalisée en une matière résorbable biologiquement une fois le filtre implanté, cette matière appartenant à la liste suivante : acide polyglycolique, acides polyactiques.

17. Filtre selon l'une quelconque des revendications 8 à 11 caractérisé en ce que le(s) filament(s) de la partie de tamisage (9,9') est (sont) réalisé(s) en une matière utilisée en chirurgie pour les sutures résorbables, tels que des filaments d'acide polyglycolique.

18. Filtre selon l'une quelconque des revendications précédentes caractérisé en ce que lesdits zigzags de la structure extérieure (7) présentent, à l'une au moins de leurs extrémités opposées (17a, 17b) où ils changent de sens, des yeux (23) pour la liaison avec ladite partie de tamisage centrale.

## Claims

1. Blood filter which can be implanted in a vessel (3) in the path of the blood, in particular in order to halt blood clots, characterised in that it comprises:
- a self-expandable outer structure (7) which contains at least one relatively rigid thread (11) in the shape of a zigzag, which is wound about itself in order to provide a closed configuration, which on the exterior forms a tubular surface which is substantially cylindrical in the expanded condition of the structure transversely to its cylinder axis;
- a central filtering part (9, 9', 9'') which extends towards the interior of this structure and is connected to the thread (11) at various points in order to halt blood clots; and
- hooking means (19) which are supported by the structure (7), to hook the filter to the vessel.

2. Blood filter according to Claim 1,
characterised in that the thread (11) in the shape of a zigzag has a series of lines (15) which are connected by curved end portions (17a, 17b), and can occupy resiliently a first, position which is folded under compression, in which these lines extend substantially side by side, one along another, in order to permit insertion of the filter in the circulatory system, and its so-called expanded position in which these lines (15) are distanced angularly from one another in order to form the substantially cylindrical tubular surface.

3. Filter according to Claim 1 or Claim 2,
characterised in that the outer structure (7) extends exclusively on the periphery of the filter, the connection between this structure and the central filtering part (9) being disposed on the periphery of the latter.

4. Filter according to any one of the preceding claims, characterised in that the central filtering part (9) is made of material which is biologically re-absorbable when the filter is implanted, the resistance to biological re-absorption of this filtering part being lower towards a substantially central area of the latter than it is at its periphery, where this filtering part is connected to the outer zigzag structure.

5. Filter according to any one of Claims 2 to 4, characterised in that the lines (15) of the outer zigzag structure are disposed such that they come substantially into contact with a wall (3a) of the vessel when the filter is implanted.

6. Filter according to any one of the preceding Claims 2 to 5, characterised in that the hooking means are provided on at least some of the lines (15) of the thread (11).

7. Filter according to Claim 6,
characterised in that the hooking means consist of hooks (19) which are attached to the lines (15) of the zigzag thread by means of plates (21), these hooks extending in pairs in opposite directions in order to prevent translation of the filter in the venous path in one direction or in the inverse direction.

8. Filter according to any one of the preceding claims, characterised in that the filtering part consists of a relatively flexible filament or series of filaments disposed in order to constitute a mesh net (9), with meshes which are smaller towards the centre than towards the periphery, where this net is connected to the zigzag thread outer structure (7).

9. Filter according to any one of Claims 1 to 7, characterised in that the filtering part (9, 9') consists of a relatively flexible filament or series of filaments with a cross-section which decreases from the periphery towards the centre of this filtering part.

10. Filter according to any one of Claims 1 to 7, characterised in that the filtering part (9') comprises a series of relatively flexible filaments (26) which extend from the interior of the zigzag thread structure, between the two opposite axial ends (17a, 17b) of the latter, at which the filaments are connected to the structure, these filaments being disposed such as to meet or overlap in the manner of a bundle, in a substantially central area (27) of the filter.

11. Filter according to Claim 10,
characterised in that between the two opposite ends (17a, 17b) of the outer structure (7), the bundle of threads (26) is completed by at least one band of a complementary thread (29) which surrounds these bundled threads (26) locally in order at least temporarily to increase the filtering power of this filtering part (9').

12. Filter according to any one of Claims 1 to 7, characterised in that the filtering part (9'') is in the shape of a single-piece star with a plurality of legs (31, 31') which are connected to one another towards the centre (33) of the star.

13. Filter according to Claim 12,
characterised in that these legs (31, 31') have a reduced cross-section locally in the vicinity of the centre (33) of the star.

14. Filter according to Claim 12 or Claim 13,
characterised in that the legs (31) have transverse branches (37) which connected two successive legs to one another, between the centre (33) and the periphery of the star.

15. Filter according to any one of Claims 12 to 14, characterised in that the star (9'') is made by being moulded, cut or stamped.

16. Filter according to any one of Claims 12 to 15, characterised in that the star (9'') is made of a substance which is biologically reabsorbable when the filter is implanted, this substance belonging to the following list: polyglycolic acid, polylactic acids.

17. Filter according to any one of Claims 8 to 11, characterised in that the filament(s) of the filtering part (9,9') is, or are, made of a substance used in surgery for re-absorbable sutures, such as polyglycolic acid filaments.

18. Filter according to any of the preceding claims, characterised in that the zigzags of the outer structure (7) have eyelets (23) on at least one of their opposite ends (17a, 17b) where they change direction, for connection with the central filtering part.

## Patentansprüche

1. Blutfilter, der in ein Gefäß (3) an der Blutbahn, insbesondere für das Zurückhalten von Blutkoagulaten implantierbar ist, dadurch gekennzeichnet, daß er aufweist:
- einen selbstdehnbaren Außenaufbau (7), der mindestens einen verhältnismäßig steifen Faden (11) aufweist, der Zickzackform hat und auf sich selbst gewickelt ist, um eine geschlossene Gestalt darzustellen, welche außen eine rohrförmige, im ausgedehnten Zustand des Aufbaues, quer zu seiner Zylinderachse im wesentlichen zylindrische Oberfläche definiert,
- eine zentrales Siebeteil (9, 9', 9''), das sich zum Inneren des Aufbaues hin erstreckt und mit dem Faden (11) an verschiedenen Punkten für das Zurückhalten eventueller Koagulate verbunden ist, und
- Festhakemittel (19), welche durch den Aufbau (7) für das Festhaken des Filters an dem Gefäß getragen werden.

2. Blutfilter nach Anspruch 1, dadurch gekennzeichnet, daß der als Zickzack ausgebildete Faden (11) eine Folge von Linien (15) aufweist, die durch gebogene Endteile (17a, 17b) verbunden sind, und elastisch eine erste unter Belastung zusammengefaltete Position einnehmen kann, in welcher sich die Linien im wesentlichen Seite an Seite längs zueinander erstrecken, um das Einführen des Filters in das Kreislaufsystem und seine ausgedehnte Position zu erlauben, bei welcher die Linien (15) winkelig zueinander im Abstand gehalten sind, um die im wesentlichen zylindrische rohrförmige Oberfläche zu bestimmen.

3. Filter nach Anspruch 1 oder Anspruch 2, dadurch gekennzeichnet, daß sich der Außenaufbau (7) ausschließlich an dem Umfang des Filters erstreckt, wobei die Verbindung zwischen diesem Aufbau und dem zentralen Siebeteil (9) an dem Umfang des letzteren angeordnet ist.

4. Filter nach einem der vorhergehenden Ansprüche, dadurch gekennzeichnet, daß das zentrale Siebeteil (9) aus biologisch resorbierbarem Material besteht, wenn der Filter implantiert ist, wobei der biologische Resorptionswiderstand dieses Siebeteils zu einer Zone (35) hin im wesentlichen zentral zu dieser viel schwächer als an seinem Umfang ist, wo das Siebeteil mit dem Außenaufbau in Zickzackform verbunden ist.

5. Filter nach einem der Ansprüche 2 bis 4, dadurch gekennzeichnet, daß die Linien (15) des Außenaufbaues in Zickzackform vorgesehen sind, um im wesentlichen mit einer Wand (3a) des Gefäßes in Kontakt zu kommen, wenn der Filter implantiert ist.

6. Filter nach einem der vorhergehenden Ansprüche 2 bis 5, dadurch gekennzeichnet, daß die Festhakemittel auf mindestens einigen der Linien (15) des Fadens (11) angeordnet sind.

7. Filter nach Anspruch 6, dadurch gekennzeichnet, daß die Festhakemittel aus Haken (19) bestehen, die an den Linien (15) des Zickzackfadens mittels Platten (21) aufgesteckt sind, wobei sich diese Haken paarweise im Gegensinn erstrecken, um die Verschiebung des Filters auf der Aderbahn in einer Richtung oder in entgegengesetzter Richtung zu verhindern.

8. Filter nach einem der vorhergehenden Ansprüche, dadurch gekennzeichnet, daß das Siebeteil von einem relativ weichen Fadenteil oder einer Serie von weichen Fadenteilen gebildet ist, der (die) angeordnet ist (sind), ein Maschennetz (9) mit Maschen zu bilden, die zur Mitte hin kleiner sind als zum Umfang hin, wo das Netz mit dem Außenaufbau (7) mit Zickzackfaden verbunden ist.

9. Filter nach einem der Ansprüche 1 bis 7, dadurch gekennzeichnet, daß das Siebeteil (9, 9'), von einem verhältnismäßig weichen Fadenteil oder einer Serie von weichen Fadenteilen ausgehend, mit einem Querschnitt gebildet ist, der sich vom Umfang zur Mitte dieses Siebeteils hin verringert.

10. Filter nach einem der Ansprüche 1 bis 7, dadurch gekennzeichnet, daß das Siebeteil (9') eine Serie von relativ weichen Fadenteilen (26) aufweist, die sich im Inneren des Aufbaues (7) mit Zickzackfaden zwischen den zwei axial entgegengesetzten Enden (17a, 17b) dieses letzteren erstrecken, gegen welche die Fadenteile mit dem Aufbau verbunden sind, wobei die Fadenteile angeordnet sind, um sich nach der Art eines Bündels in einer im wesentlichen zentralen Zone (27) des Filters zu vereinigen oder sich zu überlappen.

11. Filter nach Anspruch 10, dadurch gekennzeichnet, daß das Fadenbündel (26) zwischen den zwei entgegengesetzten Enden (17a, 17b) des Außenaufbaues (7) durch mindestens einen Ergänzungsfadenring (29) ergänzt ist, der örtlich die Bündelfäden (26) umgibt, um wenigsten vorübergehend das Filtriervermögen des Siebeteils (9') zu erhöhen.

12. Filter nach einem der Ansprüche 1 bis 7, dadurch gekennzeichnet, daß das Siebeteil (9'') die Form eines einstückigen Sterns mit mehreren Ästen (31, 31') hat, die untereinander zur Mitte (33) des Sterns hin vereinigt sind.

13. Filter nach Anspruch 12, dadurch gekennzeichnet, daß die Äste (31, 31') örtlich in der Nahe der Mitte (33) des Sterns einen verringerten Durchmesser aufweisen.

14. Filter nach Anspruch 12 oder nach Anspruch 13, dadurch gekennzeichnet, daß die Äste (31) querlaufende Abzweigungen (37) aufweisen, die untereinander die fortlaufenden Äste zwischen der Mitte (33) und dem Umfang des Sternes verbinden.

15. Filter nach einem der Ansprüche 12 bis 14, dadurch gekennzeichnet, daß der Stern (9'') durch Formarbeit, Schneidarbeit oder Lochung hergestellt ist.

16. Filter nach einem Ansprüche 12 bis 15, dadurch gekennzeichnet, daß der Stern (9''), wenn der Filter einmal implantiert ist, aus biologisch resorbierbarem Material hergestellt ist, wobei dieses Material zur folgenden Aufzählung gehört: Polyglycolsäure, Polymilchsäure.

17. Filter nach einem der Ansprüche 8 bis 11, dadurch gekennzeichnet, daß der Fadenteil (die Fadenteile) des Siebeteils (9, 9') aus einem in der Chirurgie für die resorbierbaren Nähte verwendeten Material hergestellt ist, wie zum Beispiel Polyglycolsäurefadenteile.

18. Filter nach einem der vorhergehenden Ansprüche, dadurch gekennzeichnet, daß die Zickzackformen des Außenaufbaues (7) an mindestens einem ihrer entgegengesetzten Enden (17a, 17b), wo sie die Richtung ändern, Ösen (23) für die Verbindung mit dem mittleren Siebeteil aufweisen.
